# EUROPEAN PATENT SPECIFICATION

(11) **EP 2 608 792 B1**
(45) Date of publication and mention of the grant of the patent: **11.10.2017**
(21) Application number: 11752700.2
(22) Date of filing: 24.08.2011
(51) Int. Cl.: A61K 31/517, A61K 31/5377, A61P 35/00

(54) **METHODS OF ADMINISTERING AN EGFR INHIBITOR**
VERFAHREN ZUR VERABREICHUNG EINES EGFR-HEMMERS
METHODES D'ADMINISTRATION UN INHIBITEUR D'EGFR

(30) Priority: 26.08.2010 US 377177 P
(43) Date of publication of application: 03.07.2013
(73) Proprietor: Boehringer Ingelheim International GmbH, 55216 Ingelheim am Rhein (DE)
(72) Inventor: LORENCE, Robert Michael, Ridgefield Connecticut 06877-0368 (US); SHAHIDI, Mehdi, Bracknell Berkshire RG12 8YS (GB); STOPFER, Peter, 55216 Ingelheim am Rhein (DE)
(74) Representative: Simon, Elke Anna Maria
(86) International application number: PCT/US2011/048922
(87) International publication number: WO 2012/027445

(56) References cited:
- US-A1- 2009 318 480
- CHEN H X ET AL: "Epidermal Growth Factor Receptor Inhibitors: Current Status and Future Directions", CURRENT PROBLEMS IN CANCER, MOSBY, ST LOUIS, MO, US, vol. 33, no. 4, 1 July 2009 (2009-07-01), pages 245-294, XP026803866, ISSN: 0147-0272 [retrieved on 2009-12-15]
- MINKOVSKY N ET AL: "BIBW-2992, a dual receptor tyrosine kinase inhibitor for the treatment of solid tumors", CURRENT OPINION IN INVESTIGATIONAL DRUGS, PHARMAPRESS, US, vol. 9, no. 12, 1 December 2008 (2008-12-01), pages 1336-1346, XP009129910, ISSN: 1472-4472
- MEDINA P J ET AL: "Lapatinib: A dual inhibitor of human epidermal growth factor receptor tyrosine kinases", CLINICAL THERAPEUTICS, EXCERPTA MEDICA, PRINCETON, NJ, US, vol. 30, no. 8, 1 August 2008 (2008-08-01) , pages 1426-1447, XP025430103, ISSN: 0149-2918, DOI: 10.1016/J.CLINTHERA.2008.08.008 [retrieved on 2008-08-01]
- LIN ET AL: "Drug-drug interaction mediated by inhibition and induction of P-glycoprotein", ADVANCED DRUG DELIVERY REVIEWS, ELSEVIER BV, AMSTERDAM, NL, vol. 55, no. 1, 21 January 2003 (2003-01-21), pages 53-81, XP027110081, ISSN: 0169-409X [retrieved on 2003-01-21]
- VAN ERP N P ET AL: "Clinical pharmacokinetics of tyrosine kinase inhibitors", CANCER TREATMENT REVIEWS, SAUNDERS, US, vol. 35, no. 8, 1 December 2009 (2009-12-01), pages 692-706, XP026774941, ISSN: 0305-7372 [retrieved on 2009-09-05]
- SWAISLAND H C ET AL: "PHARMACOKINETIC DRUG INTERACTIONS OF GEFITINIB WITH RIFAMPICIN, ITRACONAZOLE AND METOPROLOL", CLINICAL PHARMACOKINETICS, ADIS INTERNATIONAL LTD., AUCKLAND, NZ, vol. 44, no. 10, 1 January 2005 (2005-01-01), pages 1067-1081, XP009056412, ISSN: 0312-5963, DOI: 10.2165/00003088-200544100-00005
- CHHUN STEPHANIE ET AL: "Gefitinib-phenytoin interaction is not correlated with the C-erythromycin breath test in healthy male volunteers.", BRITISH JOURNAL OF CLINICAL PHARMACOLOGY AUG 2009 LNKD- PUBMED:19694743, vol. 68, no. 2, August 2009 (2009-08), pages 226-237, XP002666080, ISSN: 1365-2125
- COLLINS D M ET AL: "Tyrosine kinase inhibitors potentiate the cytotoxicity of MDR-substrate anticancer agents independent of growth factor receptor status in lung cancer cell lines", INVESTIGATIONAL NEW DRUGS ; THE JOURNAL OF NEW ANTICANCER AGENTS, KLUWER ACADEMIC PUBLISHERS, BO, vol. 28, no. 4, 5 June 2009 (2009-06-05), pages 433-444, XP019816878, ISSN: 1573-0646
- AGARWAL SAGAR ET AL: "Distribution of Gefitinib to the Brain Is Limited by P-glycoprotein (ABCB1) and Breast Cancer Resistance Protein (ABCG2)-Mediated Active Efflux", JOURNAL OF PHARMACOLOGY AND EXPERIMENTAL THERAPEUTICS, vol. 334, no. 1, July 2010 (2010-07), pages 147-155 URL, XP002666082, ISSN: 0022-3565
- LAACK E ET AL: "Lessons learnt from gefitinib and erlotinib: Key insights into small-molecule EGFR-targeted kinase inhibitors in non-small cell lung cancer", LUNG CANCER, ELSEVIER, AMSTERDAM, NL, vol. 69, no. 3, 1 September 2010 (2010-09-01), pages 259-264, XP027172036, ISSN: 0169-5002 [retrieved on 2010-06-19]

## Description

### BACKGROUND OF THE INVENTION

### 1. TECHNICAL FIELD

This invention relates to pharmaceutical compositions comprising BIBW 2992 or a pharmaceutically acceptable salt thereof for use in a method for treatment of a patient having non-small cell lung cancer in which there is a step of avoiding or modifying co-administration of BIBW 2992 with P-glycoprotein (P-gp) inhibitors.

Also, the invention relates to BIBW 2992 or a pharmaceutically acceptable salt thereof for use in a method for optimizing therapeutic efficacy of treatment of NSCLC in a human patient in which there is a step of reducing the dosage or dose frequency of a P-gp inhibitor or avoiding completely the administering of a P-gp inhibitor before starting of administering BIBW 2992 or a pharmaceutically acceptable salt thereof

### 2. BACKGROUND INFORMATION

Epidermal growth factor receptor (EGFR) tyrosine kinase inhibitors have been studied clinically to demonstrate efficacy in treating certain cancers. Compounds which inhibit signal transduction by tyrosine kinases, for example of the human EGF receptor, have been shown to be useful for treating pathophysiological processes caused by hyperfunction of tyrosine kinases. David W. Fry, Pharmacol. Ther. Vol. 82, Nos. 2-3, pp. 207-218, 1999*.* Several irreversible inhibitors have been shown to have therapeutic advantages such as prolonged tumor suppression compared to reversible inhibitors such as gefitinib. DeBono & Rowinsky, Br. Med. Bull. 64:227-254 (2002*).* Compounds have been disclosed in WO 02/50043, WO 2004/074263 and WO 2005/037824 as dual inhibitors of erbb1 receptor (EGFR) and erbB2 (Her2/neu) receptor tyrosine kinases, suitable for the treatment of e.g. benign or malignant tumours, particularly tumours of epithelial and neuroepithelial origin, metastasis and the abnormal proliferation of vascular endothelial cells (neoangiogenesis), for treating diseases of the airways and lungs which are accompanied by increased or altered production of mucus caused by stimulation by tyrosine kinases, as well as for treating diseases of the gastrointestinal tract and bile duct and gall bladder which are associated with disrupted activity of the tyrosine kinases.
BIBW 2992 is a highly selective, potent, irreversible tyrosine kinase inhibitor of EGFR and HER2 with promising effects having been seen in non-small cell lung cancer (NSCLC) patients. Drugs of the Future 2008, 33(8): 649-654; Li, D. et al, Oncogene (2008) 27, 4702-4711. BIBW 2992 was also reported to be effective in solid tumors besides NSCLC. Minkovsky, N. et al., Current Opinion in Investigational Drugs 2008, 9(12), 1336-1346. BIBW 2992 pharmacokinetic characteristics (PK) after single and multiple doses revealed moderately fast absorption. British Journal of Cancer (2008) 98, 80-85. BIBW 2992 exhibited more than dose-proportional increase in exposure in healthy volunteers and in cancer patients.

Skin toxicity and diarrhea were the most common adverse events in the preliminary results of a Phase II clinical trial in patients with adenocarcinoma of the lung and activating EGFR mutations. Mukherji, D., et al, Expert Opin. Investig. Drugs (2009) 18(3), 293-300.

P-gp is a transmembrane efflux pump protein and appears to be an important component of the barrier which protects tissues from potentially harmful substances by pumping them out of cells. Standard chemotherapy treatment regimens for cancer typically involve highly toxic compounds designed to kill tumor cells. In multi-drug resistance (MDR), tumor cells will use P-gp to pump out therapeutic medication before it can penetrate the tumor and effectively eliminate the cancer. Juranka PF et al., FASEB J. 1989 Dec; 3(14): 2583-92*.* While this has led to the development of agents that inhibit the action of P-gp, this approach has been unpredictable since many randomized controlled trials evaluating P-gp substrate drugs used in combination with or without P-gp modulators, have not shown significant improvements in outcome. Ferry DR et al., Eur J Cancer. 1996 Jun; 32A(6):1070-81*.* Therefore there may be other factors limiting the effectiveness of P-gp inhibitors *in vivo.* One study has shown that P-gp inhibitors effectiveness varied according to cell distances from blood vessels: P-gp overexpressing tumors had increased uptake of doxorubicin in proximal cells, minimal or no effect on drug uptake at intermediate distances from blood vessels but decreased drug uptake in more distal cells. Patel, Krupa J. et al., BMC Cancer 2009; 9: 356. Work done until now on P-gp modulators being used to increase effectiveness of chemotherapeutic drugs has shown limitations and unpredictability of such of P-gp modulators and suggest the importance of considering drug distribution in the design and development of novel treatment strategies.

### BRIEF SUMMARY OF THE INVENTION

The work cited above supports the principle that treating a patient having non-small cell lung cancer (NSCLC) with the EGFR inhibitor BIBW 2992 requires an active step of avoiding or modifying the co-administration of such drugs with P-gp inhibitors.

### DETAILED DESCRIPTION OF THE INVENTION

The present inventors have discovered for the first time that taking both drugs together, a P-gp inhibitor and an EGFR inhibitor results in an increased drug exposure of said EGFR inhibitor. The simultaneous administration of a P-gp inhibitor together with a EGFR inhibitor should be avoided and/or modified or the dose of the EGFR inhibitor should be modified.
Alternatively, administration of a potent P-gp inhibitor at least 6 hours after an EGFR inhibitor administration may minimize the DDI (drug drug interaction) which was seen during concomitant intake of EGFR inhibitor together with a P-gp inhibitor.

The invention therefore provides a pharmaceutical composition comprising BIBW 2992 or a pharmaceutically acceptable salt thereof, for use in a method of treatment of a NSCLC patient, the method comprising
(a) identifying a patient in need of treatment with BIBW 2992 or a pharmaceutically acceptable salt thereof;
(b) determining that the patient is receiving therapy with a P-gp inhibitor selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(c) causing the patient's therapy with the P-gp inhibitor to cease prior to treatment with BIBW 2992 or a pharmaceutically acceptable salt thereof;
(d) administering BIBW 2992 or a pharmaceutically acceptable salt thereof to the patient;
(e) resuming therapy with a P-gp inhibitor not earlier than 6 hours after the administration of BIBW 2992 or a pharmaceutically acceptable salt thereof.

The invention further provides a pharmaceutical composition comprising BIBW 2992 or a pharmaceutically acceptable salt thereof, for use in treating a NSCLC patient by a method comprising
(a) determining that the patient is receiving therapy with a P-gp inhibitor selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(b) reducing the dosage or dose frequency of said P-gp inhibitor before starting of administering BIBW 2992 or a pharmaceutically acceptable salt thereof; and
(c) administering BIBW 2992 or a pharmaceutically acceptable salt thereof to the patient.

In another aspect of the invention there is provided BIBW 2992 or a pharmaceutically acceptable salt thereof for use in a method of optimizing therapeutic efficacy of treatment of NSCLC in a human patient, comprising:
(a) determining that the patient is being administered an inhibitor of P-glycoprotein (P-gp) selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(b) reducing the dosage or dose frequency of said P-gp inhibitor or avoiding completely the administering of said P-gp inhibitor before starting of administering BIBW 2992 or a pharmaceutically acceptable salt thereof; and
(c) administering BIBW 2992 or a pharmaceutically acceptable salt thereof to a subject having NSCLC.

In another embodiment there is provided BIBW 2992 or a pharmaceutically acceptable salt thereof according to any of the embodiments above, wherein BIBW 2992 or a pharmaceutically acceptable salt thereof is to be administered with a dosage of about 10 to 50 mg/day, preferably about 20 to 50 mg/day, more preferably 40 mg/day, preferably in the form of a tablet, taken once daily.

In another embodiment there is provided BIBW 2992 or a pharmaceutically acceptable salt thereof according to any of the embodiments above, wherein BIBW 2992 or a pharmaceutically acceptable salt thereof is to be administered without food which shall be understood to mean at least one hour before a meal until at least 3 hours after a meal.

In another embodiment there is provided BIBW 2992 or a pharmaceutically acceptable salt thereof according to any of the embodiments above, wherein BIBW 2992 or a pharmaceutically acceptable salt thereof is formulated as a dispersible tablet/granules/pellets/powder.

In another embodiment there is provided BIBW 2992 or a pharmaceutically acceptable salt thereof according to any of the embodiments above, wherein the dispersible tablet/granules/pellets/powder is dispersible in an aqueous solvent, preferably water.

In another embodiment there is provided BIBW 2992 or a pharmaceutically acceptable salt thereof according to any of the embodiments above, wherein BIBW 2992 or a pharmaceutically acceptable salt thereof is formulated for administration orally after dispersing or dissolving said inhibitor, preferably with stirring, for at least 5, preferably at least 10, even more preferred at least 5 min in an aqueous solvent, preferably water.

In another embodiment there is provided BIBW 2992 or a pharmaceutically acceptable salt thereof according to any of the embodiments above, wherein BIBW 2992 or a pharmaceutically acceptable salt thereof is formulated for administration through a naso-gastric tube.

In another aspect of the invention there is provided BIBW 2992 or a pharmaceutically acceptable salt thereof for use in the treatment of NSCLC with a dosage regime which is adjusted according to adverse events of said EGFR inhibitor treatment, concomitant with P-gp inhibitor treatment, wherein said adverse events is monitored at a time point after the patient has initiated EGFR inhibitor treatment by measuring the amount and severity of adverse events as determined by, for example, with CTCAE version 3.0 grading (http://ctep.cancer.gov/protocoldevelopment/electronic_applications/docs/ctcaev3.pdf).

In another aspect of the invention there is provided BIBW 2992 or a pharmaceutically acceptable salt thereof according to any of the embodiments above, for use in the treatment of NSCLC wherein BIBW 2992 or a pharmaceutically acceptable salt thereof is the sole active anti-cancer ingredient.

### Definitions

All terms as used herein in this specification, unless otherwise stated, shall be understood in their ordinary meaning as known in the art. Other more specific definitions are as follows:
The terms "ErbB1", "epidermal growth factor receptor" and "EGFR" are used interchangeably herein and refer to native sequence EGFR as disclosed, for example, in Carpenter et al. Ann. Rev. Biochem. 56:881-914 (1987), including variants thereof (e.g. a deletion mutant EGFR as in Humphrey et al. PNAS (USA) 87:4207-4211 (1990)). erbB 1 refers to the gene encoding the EGFR protein product. As used herein, the EGFR protein is disclosed as GenBank accession no. NP.sub.--005219 which is encoded by the erbB1 gene, GenBank accession no. NM.sub.-005228. See also W. J. Gullick et al., 1986, Cancer Res., 46:285-292;S. Cohen et al., 1980, J. Biol. Chem., 255:4834-4842; A. B. Schreiber et al., 1983, J. Biol. Chem., 258:846-853).

EGFR inhibitor includes reversible or irreversible EGFR inhibitors.

"Reversible EGFR" inhibitors include: structural classes 4-anilinoquinazolines, 4-[aralkylamino] pyridopyrimidines, and 4-phenylaminopyrrolo-pyrimidines. See David W. Fry, Pharmacol. Ther. Vol. 82, Nos. 2-3, pp. 209-211, 1999*.* Specific examples include Gefitinib (compound ZD1839 "IRESSA"), Erlotinib (compound OSI-774, "TARCEVA"), Lapatinib, conventional cancer treatment with both IRESSA and TARCEVA involves the daily, oral administration of no more than 500 mg of the respective compounds.

"Irreversible EGFR inhibitor" includes any compound which binds irreversibly to EGFR, preferably to cysteine 773 of EGFR. Nonlimiting examples include compounds disclosed in U.S. Pat. No. 6,002,008, US 7,019,012, US 6,251,912, WO 02/50043, WO 2004/074263, WO 2005/037824, BIBW2992, EKB-569, HKI-272, HKI-357, C1-1033, Icotinib or PF-00299804, including pharmaceutical acceptable salts thereof.

Preferably BIBW 2992 dimaleate: 2-Butenamide, N-[4-[(3-chloro-4-fluorophenyl)amino]-7-[[(3S)-tetrahydro-3-furanyl]oxy]-6-quinazolinyl]-4-(dimethylamino)-, (2E)-, (2Z)-2-butendioate (1:2)

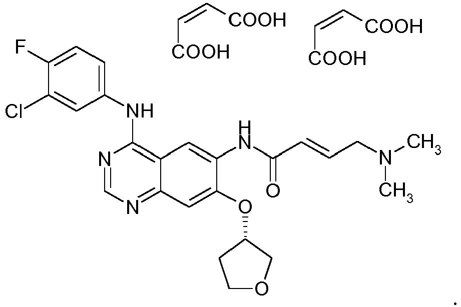

P-gp, is P-glycoprotein is encoded by the ABCB1 gene (Ueda K, Clark DP, Chen CJ, Roninson IB, Gottesman MM, Pastan I (January 1987). "The human multidrug resistance (mdr1) gene. cDNA cloning and transcription initiation". J. Biol. Chem. 262 (2): 505-8. PMID 3027054).

P-gp modulators include inhibitors as defined herein, preferably such compounds that are potent inhibitors of P-gp.

Potent P-gp inhibitors include any agent capable of preferably potently inhibiting P-gp. Nonlimiting examples include alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline and combinations thereof, Preferably Cyclosporin, Erythromycin, Ketoconazole, Itraconazole, Quinidine, Phenobarbital salt with Quinidine, Ritonavir, Valspodar or Verapamil.

"Modifying" the administering of said P-gp modulators shall be understood to mean reducing the dosage or dose frequency of said P-gp modulators.

"Avoiding" completely the administering of said P-gp modulators shall be understood to mean either a) not starting the administration of said P-gp modulators or b) stopping the administration of said P-gp modulator and then not re-starting the administration of said P-gp modulator.

The treatment may also involve a combination of treatments, including, but not limited to a tyrosine kinase inhibitor in combination with other tyrosine kinase inhibitors, chemotherapy, radiation, etc. Reference in this regard may be made to EP 09160202.9, PCT/EP/2010050338, WO 2008/121467, US 2009-0306101, US 2006-0058311, US 2005-0043233, US 2003-0225079 and US 2009-0318480.

Concomitant use of a P-gp modulator in treatment of a patient with a first medication such as an EGFR inhibitor means that the P-gp modulator is administered to the patient according to a treatment regimen characterized by repeated administration of a dosage unit in defined time intervals, e.g. once, twice or thrice daily, in addition to a parallel but independent treatment regimen characterized by repeated administration of a dosage unit of the first medication. Additionally the concomitant use of a P-gp modulator together with an EGFR inhibitor means that both drugs would be adminsitered in parallel (simultaneously) or at maximum within a time frame of 1 hour between both drug administrations.

The disclosure of WO 02/50043, WO 2004/074263 and WO 2005/037824 includes preparation as well as pharmaceutical formulations of the compounds. Furthermore, it is known for treatment of tumour diseases that the compounds may be used in monotherapy or in conjunction with other anti-tumour therapeutic agents, for example in combination with topoisomerase inhibitors (e.g. etoposide), mitosis inhibitors (e.g. vinblastine), compounds which interact with nucleic acids (e.g. cisplatin, cyclophosphamide, adriamycin), hormone antagonists (e.g. tamoxifen), inhibitors of metabolic processes (e.g. 5-FU etc.), cytokines (e.g. interferons) or antibodies.

The expression "patient" relates to a human patient suffering from cancer and thus in need of such treatment. Furthermore, the expression "patient" should be understood to include such cancer patients carrying tumors with wild-type EGF receptor as well as pre-selected cancer patients with tumors harboring activating EGFR mutations. These can be located in the tyrosine kinase domain of the EGF receptor such as for instance the L858R or L861 point mutations in the activation loop (exon 21), or in-frame deletion/insertion mutations in the ELREA sequence (exon 19), or substitutions in G719 situated in the nucleotide binding loop (exon 18). Additional activating mutations have been reported in the extracellular domain of the EGF receptor in various indications (e.g. EGFR vIII displaying exon 2-7 deletions). Other mutations such as the T790M point mutation in exon 20 as well as certain exon 20 insertions (e.g. D770_N771insNPG) which confer resistance to particular drugs should also be included, as well as double mutants such as the combined L858R / T790M mutation or the exon-19-del/T790M.

The expression "patient" should be understood to include also such cancer patients carrying tumors with wild-type HER2 receptor as well as pre-selected cancer patients with tumors harboring activating HER2 mutations, e.g. M774_A775insAYVM.

The highest dose of BIBW 2992 is 160 mg once daily for 3 days or, alternatively 100 mg once daily for 2 weeks.

The presence of specific gain-of-function mutations within the tyrosine kinase domain of the EGF receptor in a subgroup of NSCLC patients has been associated with increased sensitivity to treatment with gefitinib and erlotinib (Lynch, New England Journal Medicine 350, 2129 (2004); Paez, Science 304, 1497 (2004); Pao, Proceedings of the National Academy of Science of the United States 101, 13306 (2004)). The treatment with EGFR inhibitors may be a first line treatment, or subsequent line treatments such as cancers initially be diagnosed as gefitinib/erlotinib sensitive or predicted to be gefitinib/erlotinib sensitive by means of these methods. In particular, the L858R point mutation (exon 21) as well as deletion/insertion mutations in the ELREA sequence (exon 19) account for the majority of gefitinib responders. A secondary point mutation in exon 20, T790M, is associated with acquired resistance to gefitinib or erlotinib. This mutation is analogous to the T315I mutation identified in CML patients who relapse under imatinib treatment (imatinib resistant patients). Methods for detecting mutations in the tyrosine kinase domain of the EGF receptor are kown in the art, several corresponding diagnostic tools are approved by the FDA and commercially available, e.g. an assay for the detection of epidermal growth factor receptor mutations in patients with non-small cell lung cancer (Genzyme Corp.; see also Journal of Clinical Oncology, 2006 ASCO Annual Meeting Proceedings (Post-Meeting Edition). Vol 24, No 18S (June 20 Supplement), 2006: Abstract 10060).

Irreversible inhibitors in contrast to reversible inhibitors (e.g., gefitinib), are able to inhibit proliferation and EGF-induced EGFR phosphorylation in cell lines expressing double mutant EGF receptors (Kwak, Proceedings of the National Academy of Science of the United States 102, 7665 (2005) and Kobayashi, New England Journal Medicine 352, 786 (2005)).

Any aspect of the present invention therefore includes optional pre-selection of NSCLC patients for an EGFR mutation in the tyrosine kinase domain of the EGF receptor as well as pre-selection of NSCLC patients for an HER2 mutation. The EGFR mutations preferably relevant in in this context are selected from the group consisting of the L858R and L861 point mutations in the activation loop (exon 21), in-frame deletion/insertion mutations in the ELREA sequence (exon 19), substitutions in G719 situated in the nucleotide binding loop (exon 18), activating mutations in the extracellular domain of the EGF receptor such as EGFR vIII displaying exon 2-7 deletions, the T790M point mutation in exon 20, exon 20 insertions such as D770_N771insNPG, and double mutants such as the combined L858R / T790M mutation and the exon-19-del/T790M. The HER2 mutation preferably relevant in in this context is the M774_A775insAYVM mutation.

### Use in a method of treatment:

A method of treating NSCLC would include pre-selection of NSCLC patients for EGFR and/or HER2 mutations and administering a therapeutically effective amount of BIBW 2992 to a pre-selected NSCLC patient shown to carry an EGFR mutation in the tyrosine kinase domain of the EGF receptor and/or with a tumor harboring an activating HER2 mutation, optionally in combination with chemotherapy, biological therapy including therapeutic antibodies, targeted therapy including mTOR inhibitors, radiotherapy, radio-immunotherapy and/or tumour resection by surgery. The method further comprises modifying or avoiding completely the administering of said P-gp inhibitors before starting of administering BIBW 2992 or a pharmaceutically acceptable salt thereof.

Another method of treating NSCLC in a NSCLC patient by administering a therapeutically effective amount of BIBW 2992 to said NSCLC patient, optionally in combination with chemotherapy, biological therapy including therapeutic antibodies, targeted therapy including mTOR inhibitors, radiotherapy, radio-immunotherapy and/or tumour resection by surgery, wherein said NSCLC patient would was pre-selected based on a) having had at least 12 weeks of treatment with a reversible EGFR inhibitor and b) having failed treatment with said reversible EGFR inhibitor. The method further comprises modifying or avoiding completely the administering of said P-gp inhibitors before starting of administering BIBW 2992 or a pharmaceutically acceptable salt thereof.

BIBW 2992 can be optionally in form of its tautomers, racemates, enantiomers, diastereomers and the mixtures thereof and optionally in form of the pharmacologically acceptable acid addition salts, solvates, hydrates, polymorphs or physiologically functional derivatives thereof. BIBW 2992 is administered orally, enterically, transdermally, intravenously, peritoneally or by injection, preferably orally. In either case, the P-gp inhibitor should be avoided and/or modified.

### Dosage:

BIBW 2992 may be administered to the human patient in a daily dose of 0.01-4 mg/kg of body weight (bw), preferably 0.1-2 mg/kg, particularly preferred in a dose of 0.2-1.3 mg/kg bw. For oral treatment BIBW 2992 may be administered daily in a total dose of 10, 20, 30, 40, 50, 60, 70, 100, 200, or 300 mg, optionally divided into multiple doses, e.g. 1 to 3 doses to be administered through the day. Preferably the oral daily dose is administered only once a day. More preferably, the starting dose for BIBW 2992 is 40 mg , preferably in form of a tablet, once daily. For patients, who tolerate a 40 mg starting dose, a dose of 50 mg may be considered. For patients, who already had TKI (tyrosine kinase inhibitor) treatment, the starting dose is 50 mg, preferably in form of a tablet, once daily. Especially for higher doses periods of treatment should alternate with periods of recovery, without administering BIBW 2992. For instance, treatment could follow a "7 day on - 7 day off", a "14 day on - 14 day off", a "21 day on 7 day off" or a continuous dosing schedule. "On-off" time periods can be chosen shorter, especially if higher doses are administered, or individually adapted to the needs of the patient. Preferable dosage of BIBW 2992 is 20, 30, 40, 50mg, most preferably 40mg once daily.

The dosage for intravenous use e.g. of BIBW2992MA2 may be 1 - 1000 mg, preferably 5 - 300 mg, particularly preferred 10 - 100 mg (dosages refer to the base form BIBW2992), either given as a bolus or, especially if higher doses are applied, as a slow intravenous infusion over several hours, e.g. over about 1, 2, 4, 6, 10, 12 or 24 hours.

In one embodiment the invention relates to the use in a method of treatment described above, characterised in that BIBW 2992, or its polymorph, metabolite, hydrate, solvate, an individual optical isomer, mixtures of the individual enantiomers or racemates thereof, or a pharmaceutically acceptable salt thereof, is administered intermittent or in a daily dosage such that the plasma level of the active substance preferably lies between 10 and 5000 nM for at least 12 hours of the dosing interval.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

Moreover, the recommended intake for BIBW 2992 is without food and at least one hour before a meal or at least 3 hours after a meal.

BIBW 2992, its tautomers, the racemates, the enantiomers, the diastereomers and the mixtures thereof, and optionally the pharmacologically acceptable acid addition salts, solvates, hydrates, polymorphs, physiologically functional derivatives or prodrugs thereof, may be used in monotherapy or combined with other active substances, optionally also in conjunction with other pharmacologically active substances.

### Pharmaceutical formulations:

Suitable pharmaceutical preparations for the use in accordance with the invention include, for example, tablets, capsules, suppositories, solutions, and particularly solutions for injection (s.c., i.v., i.m.) and infusion, syrups, emulsions or dispersible powders. The amount of pharmaceutically active compound in each case should be in the range from 0.1 - 90 wt.%, preferably 0.5 - 50 wt.% of the total composition, i.e. in amounts which are sufficient to achieve the dosage range given below. The doses specified may, if necessary, be given several times a day.

Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substances or combinations thereof may additionally contain a sweetener such as saccharin, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p hydroxybenzoates. Solutions for injection and infusion are prepared in the usual way, e.g. with the addition of preservatives such as p hydroxybenzoates, or stabilisers such as alkali metal salts of ethylenediamine tetraacetic acid, optionally using emulsifiers and/or dispersants, while if water is used as the diluent organic solvents may optionally be used as solubilisers or auxiliary solvents, and transferred into injection vials or ampoules or infusion bottles.
Capsules containing one or more active substances or combinations of active substances may for example be prepared by mixing the active substances with inert carriers such as lactose or sorbitol and packing them into gelatine capsules.
Suitable suppositories may be made for example by mixing with carriers provided for this purpose, such as neutral fats or polyethyleneglycol or the derivatives thereof. Suitable excipients may be, for example, water, pharmaceutically acceptable organic solvents, such as paraffins (e.g. petroleum fractions), oils of vegetable origin (e.g. groundnut or sesame oil), mono- or polyfunctional alcohols (e.g. ethanol or glycerol), carriers such as e.g. natural mineral powders (e.g. kaolin, clays, talc, chalk), synthetic mineral powders (e.g. highly dispersed silica and silicates), sugar (e.g. glucose, lactose and dextrose), emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered in the usual way, preferably by oral or transdermal route, particularly preferably by oral route. When administered orally the tablets may, of course, contain additives, such as e.g. sodium citrate, calcium carbonate and dicalcium phosphate together with various additives, such as starch, preferably potato starch, gelatine and the like, in addition to the abovementioned carriers. Lubricants such as magnesium stearate, sodium laurylsulphate and talc may also be used to form tablets. In the case of aqueous suspensions the active substances may be combined with various flavour enhancers or colourings in addition to the abovementioned excipients.
For parenteral use, solutions of the active substances may be prepared using suitable liquid carrier materials.

Preferred formulations and drug forms for BIBW 2992 are disclosed in WO2005/037824 WO2009147238 and WO2011003853.

### Examples:

The following examples are not intended, nor are they to be construed, as limiting the invention.

### Example 1. Methods of making BIBW 2992

Methods of making BIBW 2992 can be found in US 7,019,012, WO2005/037824 and WO2007/085638.

### Example 2. Preclinical data indicated that BIBW2992 is a substrate of P-glycoprotein (P-gp)

### Passive permeability through CaCo-2 cell lavers and P-gp transport profiling:

Experiments were performed to assess its passive permeability and potential transport by P-glycoprotein (a.k.a. MDR1, ABC1) and the potential inhibition of P-gp by BIBW 2992.

B1BW 2992 exhibited high passive permeability and was a substrate of P-gp (estimated Km 10-30 uM) as well as an inhibitor of P-gp with an estimated Kᵢ of 3.4 µM (mean of two independent experiments).

### Example 3. Clinical data indicated that BIBW2992 is a substrate of P-glycoprotein (P-gp)

Since BIBW 2992 was found to be a P-gp substrate *in vitro* (see above), investigators performed a phase I trial in healthy volunteers to assess the effects of the potent P-gp inhibitor ritonavir on the pharmacokinetics (PK) of BIBW 2992 (a P-gp substrate). In this open-label, randomised, two-way crossover study the relative exposure after a single oral dose of B1BW 2992 (20 mg), co-administered with multiple oral doses of ritonavir (200 mg bid for 3 days), was compared to the exposure after a single oral dose of BIBW 2992 (20 mg) alone in healthy male volunteers. The study was designed to determine the maximum effect of P-gp inhibition on the PK of BIBW 2992.

When BIBW 2992 20 mg once daily was given in combination with ritonavir 200 mg twice daily, AUC_{0-∞} of BIBW 2992 increased by 47.6% (90% CI 133.7%, 162.9%),AUC_{0-tz} increased by 49.0% (90% CI 134.5%, 165.1 %), and Cₘₐₓ increased by38.5% (90% CI 120.6%, 158.9%) compared with BIBW 2992 given alone. This was a surprising result. Due to this relatively high concentration in the micromolar range in correlation to the average maximum BlBW 2992 plasma concentrations at steady state (I/Kᵢ <0.1), drug-drug interactions based on inhibition of P-gp by BIBW 2992 had been considered as less likely to occur.Median tₘₐₓ of BIBW 2992 was 4.00 hours with and without ritonavir. The distribution and elimination phases of BIBW 2992 appeared to be unaffected by ritonavir cotreatment. Also, the terminal half-life of BIBW 2992 was unchanged.

Since previous studies revealed that CYP3A4 enzyme-catalysed metabolic reactions play a subordinate role for the metabolism of BIBW 2992 in vivo and CYP3A4-dependent N-demethylation of BIBW 2992 was too low to be quantitatively detected in healthy volunteers, the increase in BIBW 2992 exposure in the presence of ritonavir is most likely attributed to the inhibition of P-gp-mediated transport processes during the absorption phase of BIBW 2992.

Because of these results, the following measures were implemented for clinical trials of BIBW 2992 as a protocol amendment which indicted that:
The use of potent P-gp inhibitors (including preferably Cyclosporin, Erythromycin, Ketoconazole, Itraconazole, Quinidine, Phenobarbital salt with Quinidine, Ritonavir, Valspodar, Verapamil) has to be avoided during treatment with BIBW 2992.

### Example 4. Treatment of lung cancer patients using BIBW 2992 following failure with a reversible EGFR inhibitor

Patients with adenocarcinoma of the lung who had previously failed a reversible EGFR inhibitor (e.g., gefitinib or erlotinib) and who had had at least 12 weeks of therapy with said reversible inhibitor are identified as being candidates for treatment with BIBW 2992. However, prior to treatment with BIBW 2992, certain patients are found to be taking a potent P-gp inhibitor. Prior to starting BIBW 2992 treatment, treatment with the potent P-gp inhibitor is stopped. Following this cessation of P-gp inhibitor therapy, BIBW 2992 treatment is then started at a dose of 50 mg daily.

### Example 5. BIBW 2992 treatment of lung cancer patients having tumors with EGFR mutations.

Patients with adenocarcinoma of the lung who have EGFR mutations (including, but not limited to deletion-19 mutations, other mutations in exon 19, L858R mutations, other mutations in exon 21, mutations in exon 18 and mutations in exon 20) are identified as being candidates for treatment with BIBW 2992. However, prior to treatment with BIBW 2992, certain patients are found to be taking a potent P-gp inhibitor. Prior to starting BIBW 2992 treatment, treatment with the potent P-gp inhibitor is stopped. Following this cessation of P-gp inhibitor therapy, BIBW 2992 treatment is then started at a dose of 50 mg daily.

## Claims

1. A pharmaceutical composition comprising BIBW 2992 or a pharmaceutically acceptable salt thereof, for use in a method for treatment of a non-small cell lung cancer (NSCLC) patient by a method comprising
(a) identifying a patient in need of treatment with BIBW 2992 or a pharmaceutically acceptable salt thereof;
(b) determining that the patient is receiving therapy with a P-gp inhibitor selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(c) causing the patient's therapy with the P-gp inhibitor to cease prior to treatment with BIBW 2992 or a pharmaceutically acceptable salt thereof;
(d) administering BIBW 2992 or a pharmaceutically acceptable salt thereof to the patient;
(e) resuming therapy with a P-gp inhibitor not earlier than 6 hours after the administration of BIBW 2992 or a pharmaceutically acceptable salt thereof

2. A pharmaceutical composition comprising BIBW 2992 or a pharmaceutically acceptable salt thereof, for use in a method for treatment of a non-small cell lung cancer (NSCLC) patient by a method comprising
(a) determining that the patient is receiving therapy with a P-gp inhibitor selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(b) reducing the dosage or dose frequency of said P-gp inhibitor before starting of administering BIBW 2992 or a pharmaceutically acceptable salt thereof; and
(c) administering BIBW 2992 or a pharmaceutically acceptable salt thereof to the patient.

3. The pharmaceutical composition for use in the method according to claim 1 or 2, wherein the P-gp inhibitor is selected from cyclosporin, erythromycin, ketoconazole, itraconazole, quinidine, phenobarbital salt with quinidine, ritonavir, valspodar and verapamil.

4. The pharmaceutical composition for use in the method according to claim 1, 2 or 3, wherein BIBW 2992 or a pharmaceutically acceptable salt thereof is to be administered without food which is understood to mean at least one hour before a meal until at least 3 hours after a meal.

5. BIBW 2992 or a pharmaceutically acceptable salt thereof, for use in a method for optimizing therapeutic efficacy of treatment of NSCLC in a human patient, comprising:
(a) determining that the patient is being administered an inhibitor of P-glycoprotein (P-gp) selected from alfentanil, amiloride, amiodarone, amitriptyline, astemizole, atovaquone, atorvastatin, azelastine, azidopine, azithromycin, bepridil, biricodar, bromocriptine, carbamazepine, carvedilol, chloroquine, chlorpromazine, clarithromycin, cyclosporin, cyproheptadine, darunavir, desethylamiodarone, desipramine, dexniguldipine, dexrazoxane, diltiazem, dipyridamole, disulfiram, doxazosin, elacridar, emetine, erythromycin, felodipine, fenofibrate, fentanyl, flavonoids, fluoxetine, fluphenazine, fluvoxamine, fucidin, gallopamil, glyburide, gramicidin D, grapefruit juice, garlic, green tea, haloperidol, hydrocortisone, hyroxyzine, josamycin, ketoconazole, imipramine, itraconazole, ivermectin, ketoconazole, laniquidar, lansoprazole, levothyroxin, lidocaine, loperamide, lopinavir, loratadine, lovastatin, maprotiline, mefloquine, methadone, mibefradil, midazolam, mitomycin C, nefazodone, nelfinavir, nicardipine, nitrendipine, nobiletin, norverapamil, omeprazole, orange juice, ofloxacin, paroxetine, pantoprazole, phenothiazines, phenobarbital, piperine, pimozide, probenecid, progesterone, promethazine, propafenone, propranolol, quercetin, quinacrine, quinidine, quinine, reserpine, ritonavir, saquinavir, sertraline, simvastatin, spironolactone, sufentanil, tacrolimus, tamoxifen, tariquidar, telithromycin, terfenadine, testosterone, tetrabenazine, thioridazine, trifluoperazine, trifluopromazine, trimipramine, valinomycin, vanadate, venlafaxine, verapamil, vinblastine, FK506, RU486(mifepristone), valspodar PSC 833, zosuquidar and 2n-propylquinoline or combinations thereof;
(b) reducing the dosage or dose frequency of said P-gp inhibitor or avoiding completely the administering of said P-gp inhibitor before starting of administering BIBW 2992 or a pharmaceutically acceptable salt thereof, and
(c) administering BIBW 2992 or a pharmaceutically acceptable salt thereof, to a subject having said cancer.

6. BIBW 2992 or a pharmaceutically acceptable salt thereof for use in the method according to claim 5, wherein the P-gp inhibitor is selected from cyclosporin, erythromycin, ketoconazole, itraconazole, quinidine, phenobarbital salt with quinidine, ritonavir, valspodar and verapamil.

7. BIBW 2992 or a pharmaceutically acceptable salt thereof for use in the method according to claim 5 or 6, wherein BIBW 2992 or a pharmaceutically acceptable salt thereof is to be administered without food which is understood to mean at least one hour before a meal until at least 3 hours after a meal.

## Patentansprüche

1. Pharmazeutische Zusammensetzung, umfassend BIBW 2992 oder ein pharmazeutisch akzeptables bzw. annehmbares Salz hiervon, zur Verwendung in einem Verfahren zur Behandlung von einem Patienten mit nicht-kleinzelligem Lungenkrebs (NSCLC) mit einem Verfahren, umfassend
(a) Identifizieren eines Patienten mit Bedarf für eine Behandlung mit BIBW 2992 oder einem pharmazeutisch akzeptablen bzw. annehmbaren Salz hiervon;
(b) Feststellen, ob der Patient eine Therapie mit einem P-gp-Inhibitor erhält, ausgewählt aus Alfentanil, Amilorid, Amiodaron, Amitriptylin, Astemizol, Atovaquon, Atorvastatin, Azelastin, Azidopin, Azithromycin, Bepridil, Biricodar, Bromocriptin, Carbamazepin, Carvedilol, Chloroquin, Chlorpromazin, Clarithromycin, Cyclosporin, Cyproheptadin, Darunavir, Desethylamiodaron, Desipramin, Dexniguldipin, Dexrazoxan, Diltiazem, Dipyridamol, Disulfiram, Doxazosin, Elacridar, Emetin, Erythromycin, Felodipin, Fenofibrat, Fentanyl, Flavonoiden, Fluoxetin, Fluphenazin, Fluvoxamin, Fucidin, Gallopamil, Glyburid, Gramicidin D, Grapefruitsaft, Knoblauch, grünem Tee, Haloperidol, Hydrocortison, Hyroxyzin, Josamycin, Ketoconazol, Imipramin, Itraconazol, Ivermectin, Ketoconazol, Laniquidar, Lansoprazol, Levothyroxin, Lidocain, Loperamid, Lopinavir, Loratadin, Lovastatin, Maprotilin, Mefloquin, Methadon, Mibefradil, Midazolam, Mitomycin C, Nefazodon, Nelfinavir, Nicardipin, Nitrendipin, Nobiletin, Norverapamil, Omeprazol, Orangensaft, Ofloxacin, Paroxetin, Pantoprazol, Phenothiazinen, Phenobarbital, Piperin, Pimozid, Probenecid, Progesteron, Promethazin, Propafenon, Propranolol, Quercetin, Quinacrin, Quinidin, Quinin, Reserpin, Ritonavir, Saquinavir, Sertralin, Simvastatin, Spironolacton, Sufentanil, Tacrolimus, Tamoxifen, Tariquidar, Telithromycin, Terfenadin, Testosteron, Tetrabenazin, Thioridazin, Trifluoperazin, Trifluopromazin, Trimipramin, Valinomycin, Vanadat, Venlafaxin, Verapamil, Vinblastin, FK506, RU486 (Mifepriston), Valspodar PSC 833, Zosuquidar und 2n-Propylchinolin oder Kombinationen hiervon;
(c) Veranlassen, dass die Therapie des Patienten mit dem P-gp-Inhibitor vor der Behandlung mit BIBW 2992 oder einem pharmazeutisch akzeptablen bzw. annehmbaren Salz hiervon beendet wird;
(d) Verabreichen von BIBW 2992 oder einem pharmazeutisch akzeptablen bzw. annehmbaren Salz hiervon an den Patienten;
(e) Wiederaufnehmen der Therapie mit einem P-gp-Inhibitor nicht früher als 6 Stunden nach der Verabreichung von BIBW 2992 oder einem pharmazeutisch akzeptablen bzw. annehmbaren Salz hiervon.

2. Pharmazeutische Zusammensetzung, umfassend BIBW 2992 oder ein pharmazeutisch akzeptables bzw. annehmbares Salz hiervon, zur Verwendung in einem Verfahren zur Behandlung von einem Patienten mit nicht-kleinzelligem Lungenkrebs (NSCLC) mit einem Verfahren, umfassend
(a) Feststellen, ob der Patient eine Therapie mit einem P-gp-Inhibitor erhält, ausgewählt aus Alfentanil, Amilorid, Amiodaron, Amitriptylin, Astemizol, Atovaquon, Atorvastatin, Azelastin, Azidopin, Azithromycin, Bepridil, Biricodar, Bromocriptin, Carbamazepin, Carvedilol, Chloroquin, Chlorpromazin, Clarithromycin, Cyclosporin, Cyproheptadin, Darunavir, Desethylamiodaron, Desipramin, Dexniguldipin, Dexrazoxan, Diltiazem, Dipyridamol, Disulfiram, Doxazosin, Elacridar, Emetin, Erythromycin, Felodipin, Fenofibrat, Fentanyl, Flavonoiden, Fluoxetin, Fluphenazin, Fluvoxamin, Fucidin, Gallopamil, Glyburid, Gramicidin D, Grapefruitsaft, Knoblauch, grünem Tee, Haloperidol, Hydrocortison, Hyroxyzin, Josamycin, Ketoconazol, Imipramin, Itraconazol, Ivermectin, Ketoconazol, Laniquidar, Lansoprazol, Levothyroxin, Lidocain, Loperamid, Lopinavir, Loratadin, Lovastatin, Maprotilin, Mefloquin, Methadon, Mibefradil, Midazolam, Mitomycin C, Nefazodon, Nelfinavir, Nicardipin, Nitrendipin, Nobiletin, Norverapamil, Omeprazol, Orangensaft, Ofloxacin, Paroxetin, Pantoprazol, Phenothiazinen, Phenobarbital, Piperin, Pimozid, Probenecid, Progesteron, Promethazin, Propafenon, Propranolol, Quercetin, Quinacrin, Quinidin, Quinin, Reserpin, Ritonavir, Saquinavir, Sertralin, Simvastatin, Spironolacton, Sufentanil, Tacrolimus, Tamoxifen, Tariquidar, Telithromycin, Terfenadin, Testosteron, Tetrabenazin, Thioridazin, Trifluoperazin, Trifluopromazin, Trimipramin, Valinomycin, Vanadat, Venlafaxin, Verapamil, Vinblastin, FK506, RU486 (Mifepriston), Valspodar PSC 833, Zosuquidar und 2n-Propylchinolin oder Kombinationen hiervon;
(b) Verringern der Dosis oder der Dosisfrequenz des P-gp-Inhibitors vor Beginn der Verabreichung von BIBW 2992 oder einem pharmazeutisch akzeptablen bzw. annehmbaren Salz hiervon; und
(c) Verabreichen von BIBW 2992 oder einem pharmazeutisch akzeptablen bzw. annehmbaren Salz hiervon an den Patienten.

3. Pharmazeutische Zusammensetzung zur Verwendung in dem Verfahren nach Anspruch 1 oder 2, wobei der P-gp-Inhibitor ausgewählt ist aus Cyclosporin, Erythromycin, Ketoconazol, Itraconazol, Quinidin, Phenobarbitalsalz mit Quinidin, Ritonavir, Valspodar und Verapamil.

4. Pharmazeutische Zusammensetzung zur Verwendung in dem Verfahren nach Anspruch 1, 2 oder 3, wobei BIBW 2992 oder ein pharmazeutisch akzeptables bzw. annehmbares Salz hiervon ohne Essen verabreicht wird, worunter zu verstehen ist: mindestens eine Stunde vor einer Mahlzeit bis mindestens 3 Stunden nach einer Mahlzeit.

5. BIBW 2992 oder ein pharmazeutisch akzeptables bzw. annehmbares Salz hiervon zur Verwendung in einem Verfahren zur Optimierung der therapeutischen Wirksamkeit der Behandlung von NSCLC bei einem menschlichen Patienten, umfassend:
(a) Feststellen, ob der Patient einen Inhibitor von P-Glycoprotein (P-gp) verabreicht bekommt, ausgewählt aus Alfentanil, Amilorid, Amiodaron, Amitriptylin, Astemizol, Atovaquon, Atorvastatin, Azelastin, Azidopin, Azithromycin, Bepridil, Biricodar, Bromocriptin, Carbamazepin, Carvedilol, Chloroquin, Chlorpromazin, Clarithromycin, Cyclosporin, Cyproheptadin, Darunavir, Desethylamiodaron, Desipramin, Dexniguldipin, Dexrazoxan, Diltiazem, Dipyridamol, Disulfiram, Doxazosin, Elacridar, Emetin, Erythromycin, Felodipin, Fenofibrat, Fentanyl, Flavonoiden, Fluoxetin, Fluphenazin, Fluvoxamin, Fucidin, Gallopamil, Glyburid, Gramicidin D, Grapefruitsaft, Knoblauch, grünem Tee, Haloperidol, Hydrocortison, Hyroxyzin, Josamycin, Ketoconazol, Imipramin, Itraconazol, Ivermectin, Ketoconazol, Laniquidar, Lansoprazol, Levothyroxin, Lidocain, Loperamid, Lopinavir, Loratadin, Lovastatin, Maprotilin, Mefloquin, Methadon, Mibefradil, Midazolam, Mitomycin C, Nefazodon, Nelfinavir, Nicardipin, Nitrendipin, Nobiletin, Norverapamil, Omeprazol, Orangensaft, Ofloxacin, Paroxetin, Pantoprazol, Phenothiazinen, Phenobarbital, Piperin, Pimozid, Probenecid, Progesteron, Promethazin, Propafenon, Propranolol, Quercetin, Quinacrin, Quinidin, Quinin, Reserpin, Ritonavir, Saquinavir, Sertralin, Simvastatin, Spironolacton, Sufentanil, Tacrolimus, Tamoxifen, Tariquidar, Telithromycin, Terfenadin, Testosteron, Tetrabenazin, Thioridazin, Trifluoperazin, Trifluopromazin, Trimipramin, Valinomycin, Vanadat, Venlafaxin, Verapamil, Vinblastin, FK506, RU486 (Mifepriston), Valspodar PSC 833, Zosuquidar und 2n-Propylchinolin oder Kombinationen hiervon;
(b) Reduzieren der Dosis oder der Dosisfrequenz des P-gp-Inhibitors oder vollständiges Vermeiden der Verabreichung des P-gp-Inhibitors vor Beginn der Verabreichung von BIBW 2992 oder einem pharmazeutisch akzeptablen bzw. annehmbaren Salz hiervon und
(c) Verabreichen von BIBW 2992 oder einem pharmazeutisch akzeptablen bzw. annehmbaren Salz hiervon, an einen Patienten mit dem genannten Krebs.

6. BIBW 2992 oder ein pharmazeutisch akzeptables bzw. annehmbares Salz hiervon zur Verwendung in dem Verfahren nach Anspruch 5, wobei der P-gp-Inhibitor ausgewählt ist aus Cyclosporin, Erythromycin, Ketoconazol, Itraconazol, Quinidin, Phenobarbitalsalz mit Quinidin, Ritonavir, Valspodar und Verapamil.

7. BIBW 2992 oder ein pharmazeutisch akzeptables bzw. annehmbares Salz hiervon zur Verwendung in dem Verfahren nach Anspruch 5 oder 6, wobei BIBW 2992 oder ein pharmazeutisch akzeptables bzw. annehmbares Salz hiervon ohne Essen verabreicht wird, worunter zu verstehen ist: mindestens eine Stunde vor einer Mahlzeit bis mindestens 3 Stunden nach einer Mahlzeit.

## Revendications

1. Composition pharmaceutique comprenant du BIBW 2992 ou un sel pharmaceutiquement acceptable de celui-ci, pour son utilisation dans une méthode de traitement d'un patient souffrant d'un cancer du poumon non à petites cellules (CPNPC) par une méthode comprenant
(a) l'identification d'un patient ayant besoin d'un traitement avec du BIBW 2992 ou un sel pharmaceutiquement acceptable de celui-ci ;
(b) la détermination que le patient reçoit une thérapie avec un inhibiteur de P-gp sélectionné parmi l'alfentanil, l'amiloride, l'amiodarone, l'amitriptyline, l'astémizole, l'atovaquone, l'atorvastatine, l'azélastine, l'azidopine, l'azithromycine, le bépridil, le biricodar, la bromocriptine, la carbamazépine, le carvédilol, la chloroquine, la chlorpromazine, la clarithromycine, la cyclosporine, la cyproheptadine, le darunavir, la déséthylamiodarone, la désipramine, la dexniguldipine, le dexrazoxane, le diltiazem, le dipyridamole, le disulfiram, la doxazosine, l'élacridar, l'émétine, l'érythromycine, la félodipine, le fénofibrate, le fentanyl, les flavonoïdes, la fluoxétine, la fluphénazine, la fluvoxamine, la fucidine, le gallopamil, le glyburide, la gramicidine D, le jus de pamplemousse, l'ail, le thé vert, l'halopéridol, l'hydrocortisone, l'hyroxyzine, la josamycine, le kétoconazole, l'imipramine, l'itraconazole, l'ivermectine, le kétoconazole, le laniquidar, le lansoprazole, la lévothyroxine, la lidocaïne, le lopéramide, le lopinavir, la loratadine, la lovastatine, la maprotiline, la méfloquine, la méthadone, le mibéfradil, le midazolam, la mitomycine C, la néfazodone, le nelfinavir, la nicardipine, la nitrendipine, la nobilétine, le norvérapamil, l'oméprazole, le jus d'orange, l'ofloxacine, la paroxétine, le pantoprazole, les phénothiazines, le phénobarbital, la pipérine, le pimozide, le probénécide, la progestérone, la prométhazine, la propafénone, le propranolol, la quercétine, la quinacrine, la quinidine, la quinine, la réserpine, le ritonavir, le saquinavir, la sertraline, la simvastatine, la spironolactone, le sufentanil, le tacrolimus, le tamoxifène, le tariquidar, la télithromycine, la terfénadine, la testostérone, la tétrabénazine, la thioridazine, la trifluopérazine, la trifluopromazine, la trimipramine, la valinomycine, le vanadate, la venlafaxine, le vérapamil, la vinblastine, le FK506, le RU486 (mifépristone), le valspodar PSC 833, le zosuquidar et la 2n-propylquinoléine et leurs combinaisons ;
(c) l'arrêt de la thérapie du patient avec l'inhibiteur de P-gp avant le traitement avec le BIBW 2992 ou un sel pharmaceutiquement acceptable de celui-ci ;
(d) l'administration du BIBW 2992 ou d'un sel pharmaceutiquement acceptable de celui-ci au patient ;
(e) la reprise de la thérapie avec un inhibiteur de P-gp pas avant 6 heures après l'administration du BIBW 2992 ou d'un sel pharmaceutiquement acceptable de celui-ci.

2. Composition pharmaceutique comprenant du BIBW 2992 ou un sel pharmaceutiquement acceptable de celui-ci, pour son utilisation dans une méthode de traitement d'un patient souffrant d'un cancer du poumon non à petites cellules (CPNPC) par une méthode comprenant
(a) la détermination que le patient reçoit une thérapie avec un inhibiteur de P-gp sélectionné parmi l'alfentanil, l'amiloride, l'amiodarone, l'amitriptyline, l'astémizole, l'atovaquone, l'atorvastatine, l'azélastine, l'azidopine, l'azithromycine, le bépridil, le biricodar, la bromocriptine, la carbamazépine, le carvédilol, la chloroquine, la chlorpromazine, la clarithromycine, la cyclosporine, la cyproheptadine, le darunavir, la déséthylamiodarone, la désipramine, la dexniguldipine, le dexrazoxane, le diltiazem, le dipyridamole, le disulfiram, la doxazosine, l'élacridar, l'émétine, l'érythromycine, la félodipine, le fénofibrate, le fentanyl, les flavonoïdes, la fluoxétine, la fluphénazine, la fluvoxamine, la fucidine, le gallopamil, le glyburide, la gramicidine D, le jus de pamplemousse, l'ail, le thé vert, l'halopéridol, l'hydrocortisone, l'hyroxyzine, la josamycine, le kétoconazole, l'imipramine, l'itraconazole, l'ivermectine, le kétoconazole, le laniquidar, le lansoprazole, la lévothyroxine, la lidocaïne, le lopéramide, le lopinavir, la loratadine, la lovastatine, la maprotiline, la méfloquine, la méthadone, le mibéfradil, le midazolam, la mitomycine C, la néfazodone, le nelfinavir, la nicardipine, la nitrendipine, la nobilétine, le norvérapamil, l'oméprazole, le jus d'orange, l'ofloxacine, la paroxétine, le pantoprazole, les phénothiazines, le phénobarbital, la pipérine, le pimozide, le probénécide, la progestérone, la prométhazine, la propafénone, le propranolol, la quercétine, la quinacrine, la quinidine, la quinine, la réserpine, le ritonavir, le saquinavir, la sertraline, la simvastatine, la spironolactone, le sufentanil, le tacrolimus, le tamoxifène, le tariquidar, la télithromycine, la terfénadine, la testostérone, la tétrabénazine, la thioridazine, la trifluopérazine, la trifluopromazine, la trimipramine, la valinomycine, le vanadate, la venlafaxine, le vérapamil, la vinblastine, le FK506, le RU486 (mifépristone), le valspodar PSC 833, le zosuquidar et la 2n-propylquinoléine et leurs combinaisons ;
(b) la réduction de la dose ou de la fréquence de la dose dudit inhibiteur de P-gp avant de commencer l'administration du BIBW 2992 ou d'un sel pharmaceutiquement acceptable de celui-ci ; et
(c) l'administration du BIBW 2992 ou d'un sel pharmaceutiquement acceptable de celui-ci au patient.

3. Composition pharmaceutique pour son utilisation dans la méthode selon la revendication 1 ou 2, dans laquelle l'inhibiteur de P-gp est sélectionné parmi la cyclosporine, l'érythromycine, le kétoconazole, l'itraconazole, la quinidine, un sel de phénobarbital avec la quinidine, le ritonavir, le valspodar et le vérapamil.

4. Composition pharmaceutique pour son utilisation dans la méthode selon la revendication 1, 2 ou 3, dans laquelle le BIBW 2992 ou un sel pharmaceutiquement acceptable de celui-ci doit être administré sans nourriture ce qui signifie au moins une heure avant un repas jusqu'à au moins 3 heures après un repas.

5. BIBW 2992 ou sel pharmaceutiquement acceptable de celui-ci, pour son utilisation dans une méthode d'optimisation de l'efficacité thérapeutique d'un traitement d'un CPNPC chez un patient humain, comprenant :
(a) la détermination que le patient reçoit un inhibiteur de la glycoprotéine P (P-gp) sélectionné parmi l'alfentanil, l'amiloride, l'amiodarone, l'amitriptyline, l'astémizole, l'atovaquone, l'atorvastatine, l'azélastine, l'azidopine, l'azithromycine, le bépridil, le biricodar, la bromocriptine, la carbamazépine, le carvédilol, la chloroquine, la chlorpromazine, la clarithromycine, la cyclosporine, la cyproheptadine, le darunavir, la déséthylamiodarone, la désipramine, la dexniguldipine, le dexrazoxane, le diltiazem, le dipyridamole, le disulfiram, la doxazosine, l'élacridar, l'émétine, l'érythromycine, la félodipine, le fénofibrate, le fentanyl, les flavonoïdes, la fluoxétine, la fluphénazine, la fluvoxamine, la fucidine, le gallopamil, le glyburide, la gramicidine D, le jus de pamplemousse, l'ail, le thé vert, l'halopéridol, l'hydrocortisone, l'hyroxyzine, la josamycine, le kétoconazole, l'imipramine, l'itraconazole, l'ivermectine, le kétoconazole, le laniquidar, le lansoprazole, la lévothyroxine, la lidocaïne, le lopéramide, le lopinavir, la loratadine, la lovastatine, la maprotiline, la méfloquine, la méthadone, le mibéfradil, le midazolam, la mitomycine C, la néfazodone, le nelfinavir, la nicardipine, la nitrendipine, la nobilétine, le norvérapamil, l'oméprazole, le jus d'orange, l'ofloxacine, la paroxétine, le pantoprazole, les phénothiazines, le phénobarbital, la pipérine, le pimozide, le probénécide, la progestérone, la prométhazine, la propafénone, le propranolol, la quercétine, la quinacrine, la quinidine, la quinine, la réserpine, le ritonavir, le saquinavir, la sertraline, la simvastatine, la spironolactone, le sufentanil, le tacrolimus, le tamoxifène, le tariquidar, la télithromycine, la terfénadine, la testostérone, la tétrabénazine, la thioridazine, la trifluopérazine, la trifluopromazine, la trimipramine, la valinomycine, le vanadate, la venlafaxine, le vérapamil, la vinblastine, le FK506, le RU486 (mifépristone), le valspodar PSC 833, le zosuquidar et la 2n-propylquinoléine et leurs combinaisons ;
(b) la réduction de la dose ou de la fréquence de la dose dudit inhibiteur de P-gp ou le fait d'éviter complètement l'administration dudit inhibiteur de P-gp avant de commencer l'administration du BIBW 2992 ou d'un sel pharmaceutiquement acceptable de celui-ci ; et
(c) l'administration du BIBW 2992 ou d'un sel pharmaceutiquement acceptable de celui-ci, à un sujet ayant ledit cancer.

6. BIBW 2992 ou sel pharmaceutiquement acceptable de celui-ci pour son utilisation dans la méthode selon la revendication 5, dans lequel l'inhibiteur de P-gp est sélectionné parmi la cyclosporine, l'érythromycine, le kétoconazole, l'itraconazole, la quinidine, un sel de phénobarbital avec la quinidine, le ritonavir, le valspodar et le vérapamil.

7. BIBW 2992 ou sel pharmaceutiquement acceptable de celui-ci pour son utilisation dans la méthode selon la revendication 5 ou 6, dans lequel le BIBW 2992 ou un sel pharmaceutiquement acceptable de celui-ci doit être administré sans nourriture ce qui signifie au moins une heure avant un repas jusqu'à au moins 3 heures après un repas.
